# EUROPEAN PATENT APPLICATION

(11) **EP 4 523 646 A1**
(43) Date of publication of application: **19.03.2025**
(21) Application number: 23803706.3
(22) Date of filing: 12.04.2023
(51) Int. Cl.: A61B 34/20, A61B 90/00, A61B 34/10, A61B 17/80

(54) **NAVIGATION SYSTEM FOR ORAL AND MAXILLOFACIAL SURGERY**

(30) Priority: 10.05.2022 KR 20220056997
(71) Applicant: Korea University Research and Business Foundation, Seoul 02841 (KR)
(72) Inventor: LEE, Eui Seok, Seoul 06285 (KR)
(74) Representative: IPAZ
(86) International application number: PCT/KR2023/004893
(87) International publication number: WO 2023/219282

(57) **Abstract**

The present invention relates to a navigation system for oral and maxillofacial surgery. The system may comprise: a standard tracked object mount which can be detached from the teeth of a patient and to which a standard tracked object is mounted; a bone orthopedic device comprising a plate, which includes a main body and a through-portion formed in the main body, and a locking screw, which passes through the through-portion, is inserted and fixed to the jaw bone of the patient, and can move left and right along the through-portion; a reference tracked object mount of which one side is mounted to the locking screw and the other side has a reference tracked object mounted thereto; a location tracking device that can track the standard tracked object and the reference tracked object; a storage unit that stores a 3D image of the patient captured before surgery; and a calculation unit that matches a coordinate system having the standard tracked object as the origin with a coordinate system of the 3D image, and calculates the position of the locking screw in the matched coordinate system by using the positional relationship between the standard tracked object and the reference tracked object.

## Description

### [Technical Field]

The present invention relates to an oral and maxillofacial navigation system applied to oral and maxillofacial surgery.

### [Background Art]

It is very important for a physician to provide a visibility for conditions in order to check conditions of a patient's surgical site and a surrounding area. Since applying a navigation surgical system to neurosurgical procedures in the 1980s, the navigation surgical system is currently used in fields such as neurosurgery, orthopedics, and otolaryngology. The navigation surgical system means a system that provides doctors with anatomical images of surrounding body structures, including the patient's surgical site, by utilizing MRI or CT devices, and clearly provides the doctor with a location where treatment should be performed.

In recent years, navigation surgical systems are being used in the oral and maxillofacial field for oncology surgery, orthognathic surgery, reconstructive surgery, and fracture surgery for trauma patients, which are performed through narrow oral cavities. In oral and maxillofacial surgery, which deals with the narrow and complex surgical site of the oral cavity, a main goal of surgery is to complete the planned surgery without damaging important structures in an oral cavity in a relatively narrow surgical field. Considering this, the utilization of the navigation surgical systems in the field of oral and maxillofacial surgery is expected to become more essential in the future. However, in the field of oral and maxillofacial surgery, a navigation system that calculates a real-time position of a bone fragment to be adjusted or a position of an adjustment part of a bone orthopedic device fixed to the bone fragment and displays the calculated position to the doctor in real time during surgery is rarely used. In addition, in the field of oral and maxillofacial surgery, the surgical field is relatively narrow, so it is important to accurately indicate the position, but the current navigation system is inaccurate, which greatly reduces a reliability of the current navigation system.

### [Disclosure]

### [Technical Problem]

The present invention is directed to providing a navigation system for oral and maxillofacial surgery, which calculates a real-time position of a bone fragment to be adjusted or a position of an adjustment part of a bone orthopedic device fixed to the bone fragment, and displays the calculated position to a doctor in real time during surgery.

### [Technical Solution]

In order to achieve the object, the present invention relates to a navigation system for oral and maxillofacial surgery. The system may include: a standard tracked object mount which is detachable from the teeth of a patient and to which a standard tracked object is mounted; a bone orthopedic device including a plate, which includes a main body and a through-portion formed in the main body, and a locking screw, which passes through the through-portion, is inserted and fixed to the jaw bone of the patient, and can move left and right along the through-portion; a reference tracked object mount of which one side is mounted to the locking screw and the other side has a reference tracked object mounted thereto; a location tracking device that is enabled to track the standard tracked object and the reference tracked object; a storage unit that stores a 3D image of the patient captured before surgery; and a calculation unit that matches a coordinate system having the standard tracked object as the origin with a coordinate system of the 3D image, and calculates the position of the locking screw in the matched coordinate system by using the positional relationship between the standard tracked object and the reference tracked object.

According to an exemplary embodiment, the reference tracked object may be a plurality of infrared markers, and the location tracking device may be a plurality of infrared cameras.

According to an exemplary embodiment of the present invention, the reference tracked object may be an EM pointer, and the location tracking device may include an EM generation unit and an EM tracker that tracks a location of the EM pointer.

According to an exemplary embodiment of the present invention, the navigation system for oral and maxillofacial surgery may further include a display unit displaying a plurality of partial images extracting at least a portion of the 3D image, and the storage unit may store a target arrival point of the locking screw in the partial image, and the calculation unit may calculate an arrival distance between the target arrival point and a current location of the locking screw, and the display unit may display the target arrival point and the arrival distance in real time on the partial image.

According to an exemplary embodiment of the present invention, the calculation unit may calculate an orientation of the locking screw to calculate an orientation of the bone orthopedic device on the matched coordinate system, and the display unit may display the orientation of the bone orthopedic device.

According to an exemplary embodiment of the present invention, the calculation unit may calculate a facial contour change simulation of a patient in the 3D image as the locking screw moves up to the target arrival point, and the display unit may display a 3D image to which the facial contour change simulation of the patient is reflected in real time.

According to an exemplary embodiment of the present invention, the locking screw may have a screw body and a screw head that are enabled to be coupled to each other, and an outer peripheral surface sawtooth may be formed on an outer peripheral surface of the screw head.

According to an exemplary embodiment of the present invention, the plate may include a side wall formed at one side of the main body, and a plurality of plate sawteeth may be formed at the through-portion on the side wall, the screw body may include a body portion having a screw thread formed on an outer peripheral surface thereof, and a head portion coupled onto the body portion and having a locking sawtooth formed on an outer peripheral surface thereof, and the outer peripheral surface sawtooth of the screw head may engage with the plate sawtooth, and an inner peripheral surface sawtooth engaged with the locking sawtooth of the head portion of the screw body may be formed on a portion of the inner peripheral surface of the screw head.

According to an exemplary embodiment of the present invention, the screw head may be supported on an upper surface of the main body of the plate at an upper side of the through-portion.

According to an exemplary embodiment of the present invention, the inner peripheral surface of the screw head may include a locking area in which the inner peripheral surface sawtooth is formed, and a locking release area in the locking area, and the locking area may be formed to extend at a predetermined height in the inner peripheral surface of the screw head.

According to an exemplary embodiment of the present invention, a catching jaw protruding inward may be formed on a lower end of the inner peripheral surface of the screw head, and a lower surface of the head portion of the screw body may be enabled to be caught on the catching jaw.

According to an exemplary embodiment of the present invention, the navigation system for oral and maxillofacial surgery may further include an elastic member which is placed between the lower surface of the screw head, and the main body, and of which one side is bent toward the lower surface of the screw head.

According to an exemplary embodiment of the present invention, the elastic member may be a spring washer.

According to an exemplary embodiment of the present invention, the navigation system for oral and maxillofacial surgery may further include a head ratchet sawtooth formed on the lower surface of the screw head, and a body ratchet sawtooth formed on an upper surface of the main body and engaged with the head ratchet sawtooth.

According to an exemplary embodiment of the present invention, the locking screw may include a first locking screw, and a second locking screw inserted into the through-portion and placed to be spaced apart from the first locking screw.

According to an exemplary embodiment of the present invention, a screw fixing hole spaced apart from the through-portion and inserted with a screw may be formed on the plate.

According to an exemplary embodiment of the present invention, the plate may include a first support portion and a second support portion which protrude from a bottom surface of the main body, and are spaced from each other, and include a worm supported by the first support portion and the second support portion, and engaged with the outer peripheral surface sawtooth of the locking screw by using the locking screw as a worm heel.

According to an exemplary embodiment of the present invention, one end portion of the worm may be supported by passing through the first support portion, and the other end portion of the worm may be supported by passing through the second support portion.

According to an exemplary embodiment of the present invention, the navigation system for oral and maxillofacial surgery may further include a motor connected to the worm, and rotating the worm in one direction or the other direction.

According to an exemplary embodiment of the present invention, the navigation system for oral and maxillofacial surgery may further include a display unit displaying a plurality of partial images extracting at least a portion of the 3D image, and the storage unit may store a predetermined target arrival point among the partial images, the calculation unit may calculate an arrival distance between the target arrival point and the locking screw, and the display unit displays the target arrival point and the arrival distance on the partial image, and the motor may rotate the worm so that the locking screw moves up to the target arrival point.

According to an exemplary embodiment of the present invention, the plate further includes a side wall formed at the other side of the main body, and a plurality of plate sawteeth engaged with the outer peripheral surface sawtooth of the locking screw may be formed at an opposite side to the worm on the side wall.

According to an exemplary embodiment of the present invention, the navigation system for oral and maxillofacial surgery may further include a second locking screw inserted into the through-portion, and placed to be spaced apart from the locking screw, and the second locking screw may be enabled to move left and right along the through-portion by engaging with the plate sawtooth.

### [Advantageous Effects]

According to the present invention, it is possible to calculate a real-time position of a bone fragment to be adjusted or a position of an adjustment part of a bone orthopedic device fixed to the bone fragment, and display the calculated position to a doctor in real time during surgery.

In addition, since a standard tracked object mount that is fitted onto the patient's teeth is used, there is almost no shaking, so the standard tracked object mount can be accurately tracked with an infrared camera or EM tracker.

In addition, a current real-time position of the bone fragment or arrival distance information of an adjustment part of a bone orthopedic device fixed to the bone fragment to a target arrival point, and current orientation information of the bone fragment or the bone orthopedic device can be displayed to a doctor.

In addition, according to an exemplary embodiment, a locking screw can be moved to a target arrival point through a worm gear, and as the locking screw moves to the target arrival point, a three-dimensional image reflecting a simulation of the patient's facial contour is displayed on the screen in real time, so that the doctor and the patient can decide on the direction of surgery or re-surgery while looking at a screen together.

### [Description of Drawings]

FIG. 1 is a diagram illustrating an overall configuration of a navigation system for oral and maxillofacial surgery according to the present invention.
FIG. 2 is a diagram illustrating a standard tracked object mount fitted to a patient's teeth in FIG. 1, and a plurality of infrared cameras tracking the standard tracked object mount.
FIG. 3 is a perspective view illustrating a state in which a locking screw is mounted on a plate in a bone orthopedic device with adjustable spacing according to a first exemplary embodiment of the present invention.
FIG. 4 is an exploded view of the bone orthopedic device with adjustable spacing of FIG. 3.
FIG. 5 is a perspective view illustrating a locking state in which the screw body engages with the screw head in the locking screw of FIG. 3.
FIG. 6 is a perspective view illustrating a locking release state in which the state in which the screw body engages with the screw head is released in FIG. 5.
FIG. 7 is a plan view illustrating an operation state of the bone orthopedic device with adjustable spacing according to the first exemplary embodiment of the present invention.
FIG. 8 is a perspective view illustrating an exemplary embodiment in which an elastic member for assisting a raising force of a screw head is additionally mounted on the bone orthopedic device with adjustable spacing in FIG. 3.
FIG. 9 is a perspective view illustrating an application example of applying a ratchet scheme to the bone orthopedic device with adjustable spacing in FIG. 3.
FIG. 10 is a plan view illustrating an exemplary embodiment in which the bone orthopedic device with adjustable spacing in FIG. 3 includes two locking screws.
FIG. 11 is a plan view illustrating an application example in which a separate screw fixing hole is formed in the bone orthopedic device with adjustable spacing in FIG. 3.
FIG. 12 is a perspective view illustrating a state in which a locking screw engages with a worm in a bone orthopedic device with adjustable spacing according to a second exemplary embodiment of the present invention.
FIG. 13 is an exploded view of the bone orthopedic device with adjustable spacing in FIG. 12.
FIG. 14 is a perspective view illustrating a state in which a second side wall is provided in the bone orthopedic device with adjustable spacing according to the second exemplary embodiment of the present invention.
FIG. 15 is a plan view illustrating an application example, in which the bone orthopedic device with adjustable spacing in FIG. 14 includes the locking screw and the second locking screw.

### [Best Mode]

Hereinafter, specific contents for exemplary embodiments of the present invention will be described in detail with reference to the accompanying drawings. In addition, in describing the present invention, a detailed description of related known functions will be omitted if it is determined that they unnecessarily make the gist of the present invention unclear.

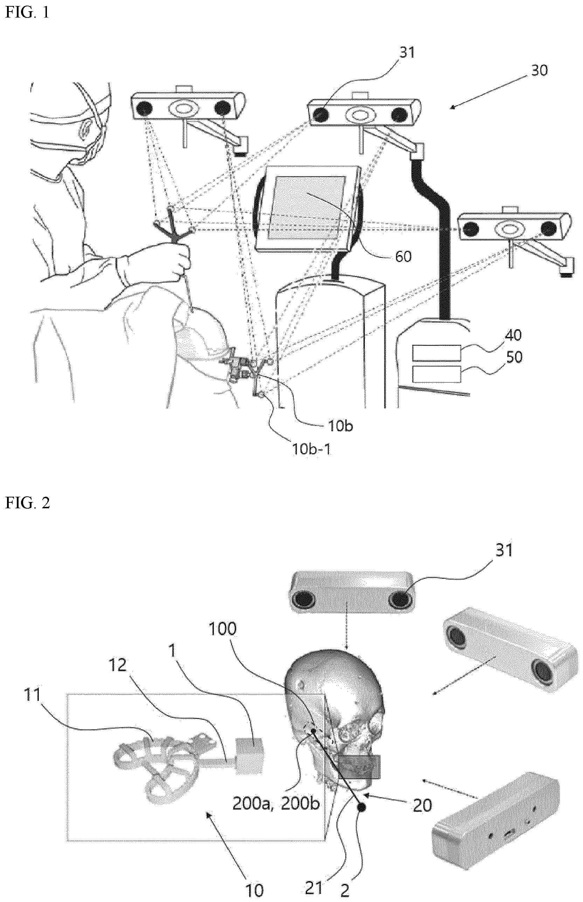

FIG. 1 is a diagram illustrating an overall configuration of a navigation system for oral and maxillofacial surgery according to the present invention. FIG. 2 is a diagram illustrating a standard tracked object mount fitted to a patient's teeth in FIG. 1, and a plurality of infrared cameras tracking the standard tracked object mount.

Referring to FIGS. 1 and 2, the oral and maxillofacial surgery navigation system according to the present invention includes a standard tracked object mount 10 (illustrated in FIG. 2), a bone orthopedic device 100, a reference tracked object mount 20, a location tracking device 30, a storage unit 40, a calculation unit 50, and a display unit 60.

The standard tracked object mount 10 is provided in a detachable form on the patient's teeth as illustrated in FIG. 2, and a standard tracked object 1 is mounted thereon. The standard tracked object mount 10 includes a mouthpiece-type tooth mounting part 11 so that one side may be fitted onto the patient's teeth, and a standard tracked object fixing member 12 extending from the tooth mounting part 11 to the other side. The standard tracked object 1 is mounted on an end of the standard tracked object fixing member 12. The standard tracked object 1 may be formed of a plurality of infrared markers or EM pointers (not illustrated). The tooth mounting part 11 of the standard tracked object mount 10 is fitted to the patient's teeth in the form of a mouthpiece, so there is almost no shaking, and it is possible to accurately track the standard tracked object 1 with an infrared camera 31 or an EM tracker (not illustrated). Meanwhile, a second standard tracked object mount 10b for accurate registration may also be additionally placed on the patient's head. According to an exemplary embodiment, the second standard tracked object mount 10b may also include an infrared marker 10b-1 or an EM pointer (not illustrated). More accurate registration may be performed by matching a coordinate system of the standard tracked object mount 10 and a coordinate system of the second standard tracked object mount 10b with each other.

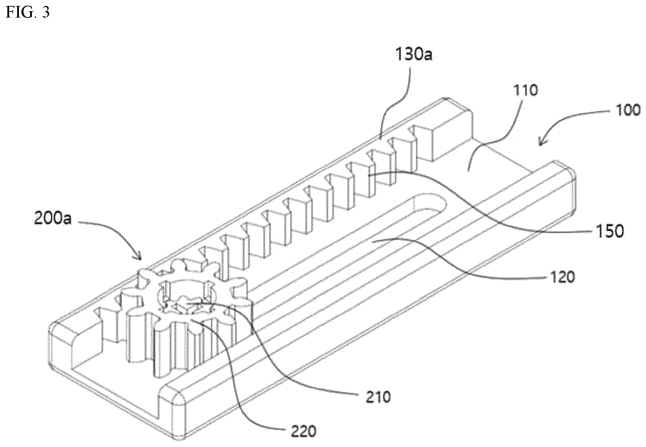

FIGS. 3 to 12 are drawings illustrating various exemplary embodiments of the bone orthopedic device 100 of the present invention. Referring to FIGS. 3 to 12, the bone orthopedic device 100 of the present invention includes a main body 110, a plate 100 including a through-portion 120 formed in the main body 110, and locking screws 200a and 200b that are inserted and fixed into the patient's jaw bone through the through-portion 120 and are movable left and right along the through-portion 120. The locking screw 200a and the locking screw 200b are screws fixed to one of the two bone fragments, and correspond to locking screws according to different exemplary embodiments. A detailed configuration of the bone orthopedic device 100 will be described later.

One side of the reference tracked object mount 20 is mounted on the locking screws 200a and 200b, and a reference tracked object 2 is mounted on the other side of the reference tracked object mount 20. The reference tracked object mount 20 includes a reference tracked object fixing member 21 of which one side is fixed to the locking screws 200a and 200b, and the other side is extended outside the patient's oral cavity. The reference tracked object 2 is mounted on an end of the reference tracked object fixing member 21. The reference tracked object 2 may be formed of a plurality of infrared markers or EM pointers (not illustrated).

The location tracking device 30 is designed to be able to track the standard tracked object mount 10 and the reference tracked object mount 20. The location tracking device 30 may be composed of the plurality of infrared cameras 31, or a module including an EM generation unit, and an EM tracker (not illustrated) tracking a position of the EM pointer. The plurality of infrared cameras 31 may be constituted by stereo cameras which are oriented toward the patient differently.

The storage unit 40 stores a 3D image of the patient photographed before surgery. Before surgery, a three-dimensional image of the patient's internal skeleton is photographed using a CT image or MRI image. The captured 3D image is transmitted to and stored in the storage unit 40.

The calculation unit 50 matches a coordinate system with the standard tracked object 1 as the origin and a coordinate system of the 3D image. Next, using a positional relationship between the standard tracked object 1 and the reference tracked object 2, the positions of the locking screws 200a and 200b are calculated within the matched coordinate system. Meanwhile, since known matching algorithms may be applied to coordinate system matching and necessary operations, a related description is omitted.

The display unit 60 displays a plurality of partial images which extracts at least a part of the 3D image of the patient obtained from the MRI or CT. Target arrival points of the locking screws 200a and 200b within the partial image are stored in the storage unit 40. The target arrival point may be determined in advance through a simulation from the 3D image of the patient before surgery. The calculation unit 50 may calculate arrival distances between the target arrival point, and current positions of the locking screws 200a and 200b, and the display unit 60 may display the target arrival point and the arrival distance on the partial image in real time. Further, the calculation unit 50 may calculate an orientation of the bone orthopedic device 100 on the matched coordinate systems by calculating orientations of the locking screws 200a and 200b, and display the calculated orientation of the bone orthopedic device 100.

According to an exemplary embodiment, the calculation unit 50 may calculate a facial contour change simulation of the patient in the 3D image as the locking screws 200a and 200b move up to the target arrival points, and the display unit 60 may display a 3D image to which the facial contour change simulation of the patient is reflected in real time. Accordingly, the doctor and the patient may determine a direction of surgery or reoperation by looking at a facial contour simulation of the patient together.

Hereinafter, each component of the bone orthopedic device 100 applied to the present invention will be described in detail.

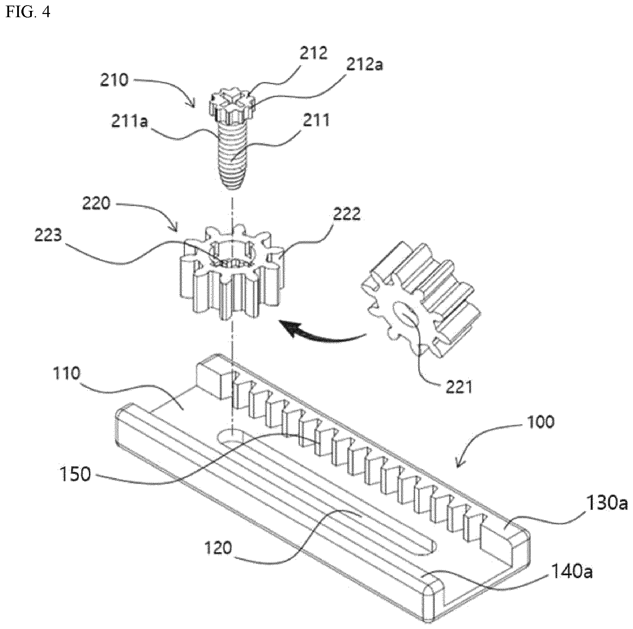

FIG. 3 is a perspective view illustrating a state in which a locking screw is mounted on a plate in a bone orthopedic device with adjustable spacing according to a first exemplary embodiment of the present invention. FIG. 4 is an exploded view of the bone orthopedic device with adjustable spacing of FIG. 3.

Referring to FIGS. 3 and 4, the bone orthopedic device with adjustable spacing according to the first exemplary embodiment of the present invention includes a plate 100 and a locking screw 200a.

The plate 100 includes a main body 110, a through-portion 120 formed to pass through a center of the main body 110 with a predetermined length, and a side wall 130a formed on one side of the main body 110. On the side wall 130a, a plurality of plate sawteeth 150 is formed to extend in a longitudinal direction of the side wall 130a toward the through-portion 120.

The plate 100 is placed across two adjacent bone fragments, a first bone and a second bone, and one surface of the main body 110 of the plate 100 is supported on an upper surface of the first bone and the other surface is supported on an upper surface of the second bone. Since the first bone and the second bone are spaced apart from each other, a predetermined area of the center of the main body 110 becomes an area which is not in contact with either the first bone or the second bone.

The through-portion 120 has an oval shape that extends in the longitudinal direction of the main body 110. The through-portion 120 is spaced apart from the side wall 130a by a predetermined spacing and is oriented parallel to the side wall 130a. One side of the through-portion 120 is placed at an upper portion of the first bone, and the other side is placed at an upper portion of the second bone. A width of the through-portion 120 is formed to be larger than a diameter of a body portion 211 of a screw body 210, while being formed to be smaller than a diameter of a head portion 212 of the screw body 210.

A plurality of plate sawteeth 150 is formed on the side wall 130a, and a second side wall 140a is formed on the other side of the plate 100 facing the side wall 130a. The side wall 130a and the second side wall 140a are formed to extend upward from one end portion and the other end portion of the plate, respectively. A spacing between longitudinal axial lines of the second side wall 140a and the through-portion 120 is formed to be larger than a spacing between longitudinal axial lines of the side wall 130a and the through-portion 120. A distance between the longitudinal axial lines of the side wall 130a and the through-portion 120 is formed approximately at a radius of a screw head 220 of the locking screw 200a. A height of each of the side wall 130a and the second side wall 140a may be formed to be equal to a height of the screw head 220.

The plate sawtooth 150 is formed to extend in a longitudinal direction of an inner surface of the side wall 130a with a length larger than a length of the through-portion 120. An extended height of the plate sawtooth 150 may be formed to be equal to an extended height of an outer peripheral sawtooth 222 formed on an outer peripheral surface of the screw head 220.

The locking screw 200a is inserted into the through-portion 120. The locking screw 200a includes the screw body 210 and the screw head 220 which are enabled to be coupled to each other. In the screw head 220, a larger hollow than the diameter of the head portion 212 of the screw body 210 is formed, so the screw body 210 may be inserted into the screw head 220. In a state where the screw head 220 is fixed on the main body 110 of the plate 100 on an upper side of the through-portion 120, the screw body 210 may be inserted into the first or second bone by passing through the through-portion 120 and be coupled into the inside of the hollow of the screw head 220.

The screw body 210 includes a body portion 211 having a screw thread 211a formed on a lower outer peripheral surface of the screw body 210, and a head portion 212 having a locking sawtooth 212a formed on an upper outer peripheral surface of the screw body 210. While the body portion 211 of the screw body 210 passes through the through-portion 120 and is inserted into the first or second bone, the screw thread 211a formed in the body portion 211 has a screw groove corresponding to the screw thread 211a in the first or second bone and engages with the screw thread 211a. An insertion groove 212b for inserting a driver, etc., is formed on an upper surface of the head portion 212 of the screw body 210. A user inserts the driver, etc., into an insertion groove 212b, and rotates the driver, etc., to insert the screw body 210 into the first or second bone.

The screw head 220 is supported on an upper surface of the main body 110 of the plate 100 on an upper side of the through-portion 120. The screw head 220 may be placed between the side wall 130a and the second side wall 140a, and may be formed at the same height as the side wall 130a and the second side wall 140a.

A catching jaw 221 protruding inward is formed at a lowest adjacent portion of an inner peripheral surface of the screw head 220. While the screw body 210 is inserted into the first or second bone, a lower surface of the head portion 212 of the screw body 210 is caught in the catching jaw 221 of the screw head 220. The head portion 212 of the screw body 210 is supported on the catching jaw 221, while the catching jaw 221 extends with a length not to be in contact with the body portion 211 of the screw body 210. The head portion 212 of the screw body 210 is caught on the catching jaw 221 not to be inserted downward any further.

In an outer peripheral surface of the screw head 220, an outer peripheral surface sawtooth 222 corresponding to a shape of the plate sawtooth 150 is formed. The outer peripheral surface sawtooth 222 is formed along a circumferential surface of the screw head 220. The outer peripheral surface sawtooth 222 is configured to move at a gear ratio set in a longitudinal direction of the through-portion 120 when the screw head 220 rotates with engaging with the plate sawtooth 150.

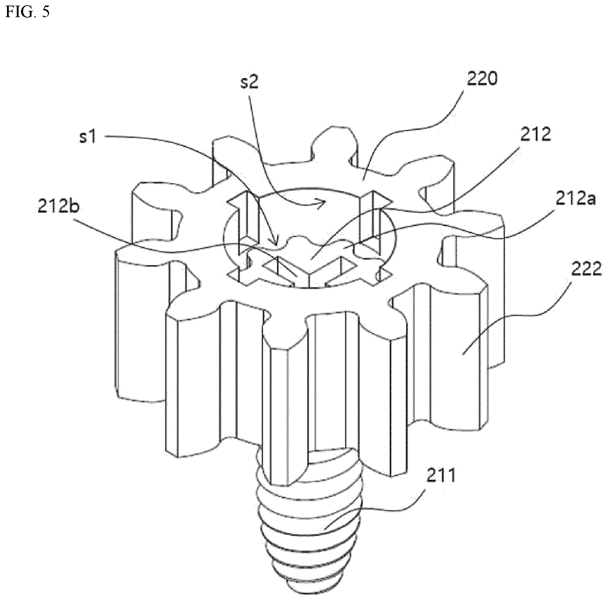

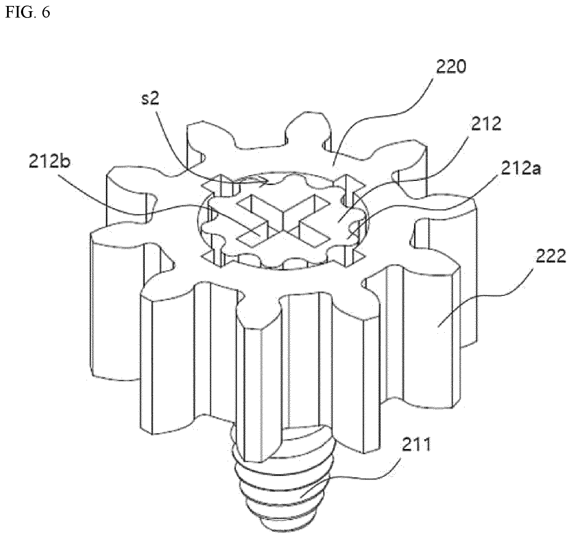

FIG. 5 is a perspective view illustrating a locking state in which the screw body engages with the screw head in the locking screw of FIG. 3. FIG. 6 is a perspective view illustrating a locking release state in which the state in which the screw body engages with the screw head is released in FIG. 5.

Referring to FIGS. 5 and 6, an inner peripheral surface sawtooth 223 corresponding to the shape of the locking sawtooth 212a of the head portion 212 of the screw body 210 is formed on a portion of the inner peripheral surface of the screw head 220. The inner peripheral surface sawtooth 223 is formed along a circumference of a portion of the inner peripheral surface of the screw head 220. The inner peripheral surface sawtooth 223 may be formed at a lower area of the inner peripheral surface of the screw head. An extended height of the inner peripheral surface sawtooth 223 may be formed to be equal to an extended height of the locking sawtooth 212a of the head portion 212. The inner peripheral surface sawtooth 223 is formed to engage with the locking sawtooth 212a of the head portion 212 of the screw body 210 while the screw head 220 ascends or descends with respect to the screw body 210.

In the inner peripheral surface of the screw head 220, an area where the inner peripheral surface sawtooth 223 is formed is referred to as a locking area s1, and an area other than the locking area s1 is referred to as a locking release area s2. The inner peripheral surface sawtooth 223 is formed only at the locking area s1 of a portion of the inner peripheral surface of the screw head 220. As a result, as a height of the screw body 210 is changed in a hollow of the screw head 220, a locking state in which the inner peripheral surface sawtooth 223 and the locking sawtooth 212a engage with each other or a locking release state in which the inner peripheral surface sawtooth 223 and the locking sawtooth 212a do not engage with each other may be selectively formed. Meanwhile, a plurality of rotating grooves 225 into which an insertion mechanism (not illustrated) is inserted is formed on an inner peripheral surface on the locking release area s2 of the screw head 220 in a longitudinal direction of the plurality of rotating grooves 225. The insertion mechanism is inserted into the rotating groove 225, and is pressed to lower the screw head 220. Further, the insertion mechanism is inserted into the rotating groove 225, and is rotated to rotate the screw head 220.

When the inner peripheral surface sawtooth 223 is formed at a lower portion of the inner peripheral surface of the screw head 220, a locking state in which the locking sawtooth 212a of the screw body 210 engages with the inner peripheral surface sawtooth 223 of the screw head 220 is formed while the screw head 220 ascends. Meanwhile, a locking release state is formed which deviates from the state in which the locking sawtooth 212a of the screw body 210 engages with the inner peripheral surface sawtooth 223 of the screw head 220 while the screw head 220 descends.

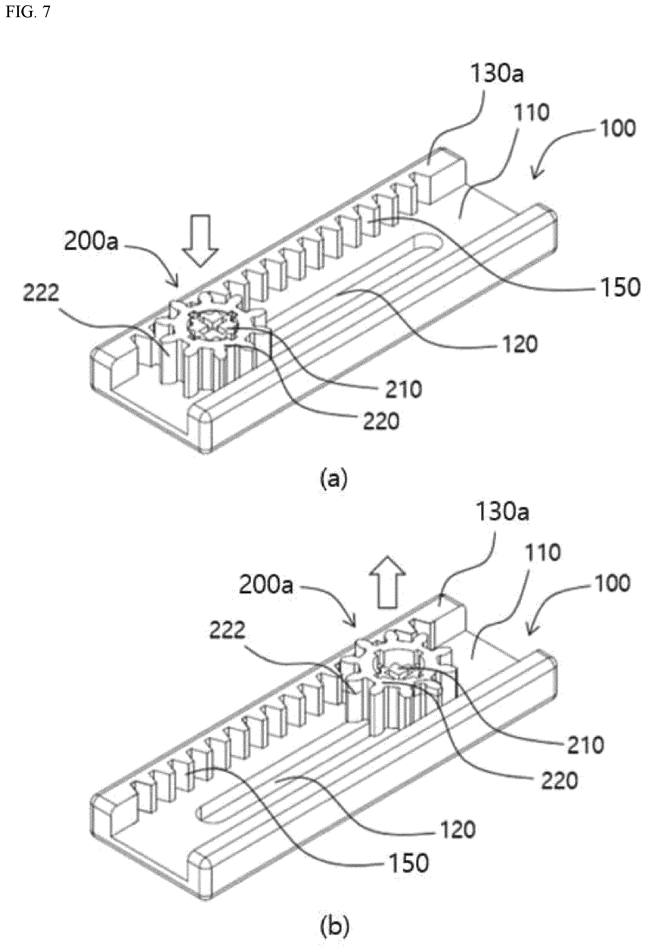

FIG. 7 is a plan view illustrating an operation state of the bone orthopedic device with adjustable spacing according to the first exemplary embodiment of the present invention.

Referring to FIG. 7, a procedure using the bone orthopedic device with adjustable spacing according to the first exemplary embodiment of the present invention includes a preparation step, a screw body insertion step, a screw head pressing step, a screw head rotation step, and a screw head raising step. Up-down and rotational movements are selectively applied to a screw head 220. At this time, an operator may directly press or rotate the screw head 220 using a tool, etc., or the up-and-down movement and rotational movement of the screw head 220 may be controlled by a driving unit including a motor.

In the preparation step, the operator places a plate 100 across a first bone and a second bone, such that one side of the plate 100 contacts a surface of the first bone and the other side of the plate 100 contacts an upper surface of the second bone. At this time, the screw head 220 is mounted on the plate 100.

In the screw body insertion step, the operator inserts and fixes a body portion 211 of a screw body 210 into the first or second bone by passing the screw body 210 through a hollow portion of the screw head 220 and a through-portion 120 of the plate 100. At this time, the head portion 212 of the screw body is caught on a catching jaw 221, and fixed.

When an inner peripheral surface sawtooth 223 is formed at a lower portion of an inner peripheral surface of the screw head 220, a locking state in which a locking sawtooth 212a of the screw body 210 engages with the inner peripheral surface sawtooth 223 of the screw head 220 is formed while the screw body 210 descends inside a hollow of the screw head 220 (see FIG. 5).

In the screw head pressing step, while the screw head 220 descends, the head portion 212 of the screw body 210 is positioned at an upper portion of the hollow of the screw head 220. Accordingly, a locking release state is formed in which the engagement between the locking teeth 212a of the screw body 210 and the inner peripheral surface sawteeth 223 of the screw head 220 is released (see FIG. 6).

In the screw head rotation step, the screw head 220 rotates in one direction or the other direction and moves to one side or the other side of a longitudinal direction of the through-portion 120. The screw body 210 inserted into the hollow of the screw head 220 is disengaged from the screw head 220, but moves together with the movement of the screw head 220. A spacing between the first bone and the second bone is adjusted in such a scheme in which a distance between the first bone and the second bone becomes closer or farther apart depending on a rotational direction of the screw head 220.

In the screw head raising step, the screw head 220 may be raised naturally due to an inherent elasticity of the bone itself. Accordingly, while the locking sawtooth 212a of the screw body 210 engages with the inner peripheral surface sawtooth 223 of the screw head 220, the locking release state is returned to the locking state. In the locking state, the screw body 210 is fixed to the first bone or the second bone, so the screw head 220 engaged with the screw body 210 does not move easily along a plate sawtooth 150 of the plate 100. Due to the inherent elasticity of the bone, the screw head 220 may be raised at approximately 0.5 mm to 3 mm.

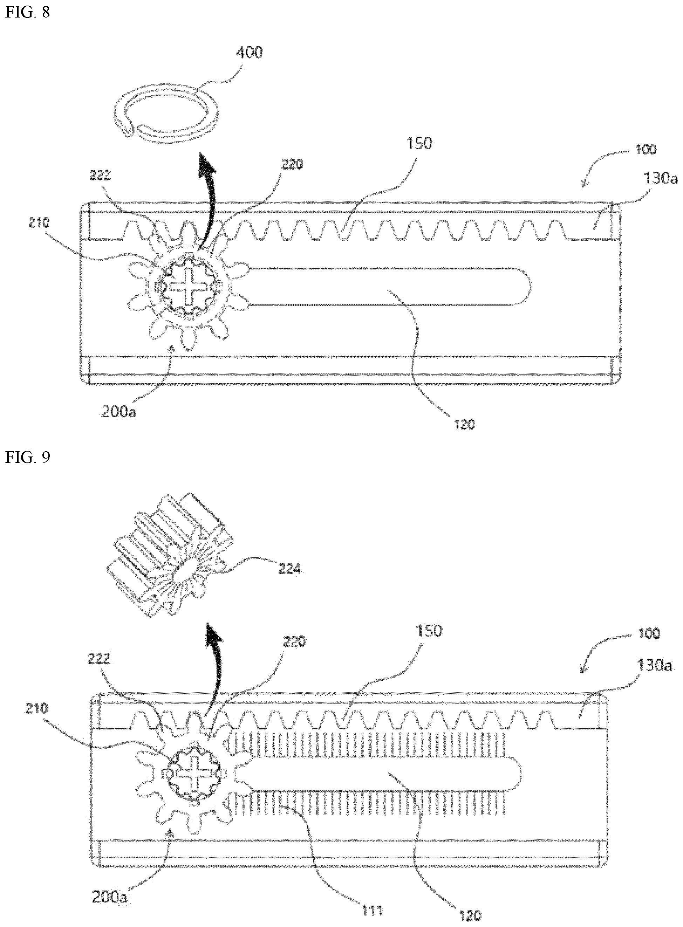

FIG. 8 is a perspective view illustrating an exemplary embodiment in which an elastic member for assisting a raising force of a screw head is additionally mounted on the bone orthopedic device with adjustable spacing in FIG. 3.

Referring to FIG. 8, a bone orthopedic device with adjustable spacing according to another exemplary embodiment of the present invention may include an elastic member 400 which is disposed between a lower surface of the screw head 220 and a main body 110, and has one side inclined toward the lower surface of the screw head 220. The elastic member 400 may be a washer, which may be a spring washer that extends obliquely from one end to the other end, such that heights of the one end and the other end are different from each other. Accordingly, the elastic member 400 may provide an additional raising force to the inherent elasticity of the bone itself.

FIG. 9 is a perspective view illustrating an application example of applying a ratchet scheme to the bone orthopedic device with adjustable spacing in FIG. 3.

Referring to FIG. 9, the bone orthopedic device with adjustable spacing according to the first exemplary embodiment of the present invention may include a head ratchet sawtooth 224 formed on the lower surface of the screw head 220, and a body ratchet sawtooth 111 formed on an upper surface of the main body 110 and corresponding to a shape of the head ratchet sawtooth 224. While the screw head 220 rotates, the head ratchet sawtooth 224 of the screw head 220 engages with a body ratchet sawtooth 111 of the main body 110. Accordingly, a 'click' sound is generated whenever the screw head 220 rotates, and a frictional force may be not only provided to precisely control the rotation of the screw head 220, but the screw head 220 may be also prevented from idling on the main body 110 when stopped.

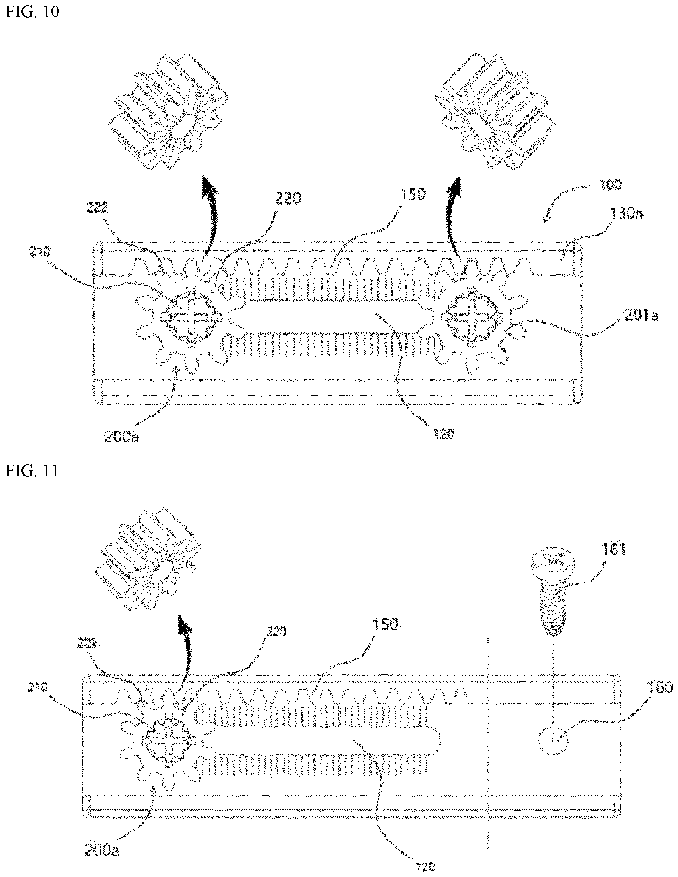

FIG. 10 is a plan view illustrating an exemplary embodiment in which the bone orthopedic device with adjustable spacing in FIG. 3 includes two locking screws.

Referring to FIG. 10, the bone orthopedic device with adjustable spacing according to another exemplary embodiment of the present invention may include a first locking screw 200a and a second locking screw 201a. The second locking screw 201a is placed within the through-portion 120, and is placed to be spaced apart from the first locking screw 200a in the longitudinal direction of the through-portion 120. The first locking screw 200a is placed on an upper side of the first bone, and the second locking screw 201a is placed on an upper side of the second bone. Through this, while the second locking screw 201a is fixed in position relative to the second bone, the movement of the first bone may be controlled by operating the first locking screw 200a, and while the first locking screw 200a is fixed in position relative to the first bone, the movement of the second bone may be controlled by operating the second locking screw 201a.

FIG. 11 is a plan view illustrating an application example in which a separate screw fixing hole is formed in the bone orthopedic device with adjustable spacing in FIG. 3.

Referring to FIG. 11, on the plate 100 of the bone orthopedic device with adjustable spacing according to another exemplary embodiment of the present invention, a screw fixing hole 160 spaced apart from the through-portion 120 may be formed. A locking screw 200a may be operated while the plate 100 is fixed to the first or second bone using a fixing screw 161 in the screw fixing hole 160. Accordingly, a fixing force of the plate 100 is increased, enabling a more stable procedure.

A second exemplary embodiment of the bone orthopedic device with adjustable spacing in the present invention is described below.

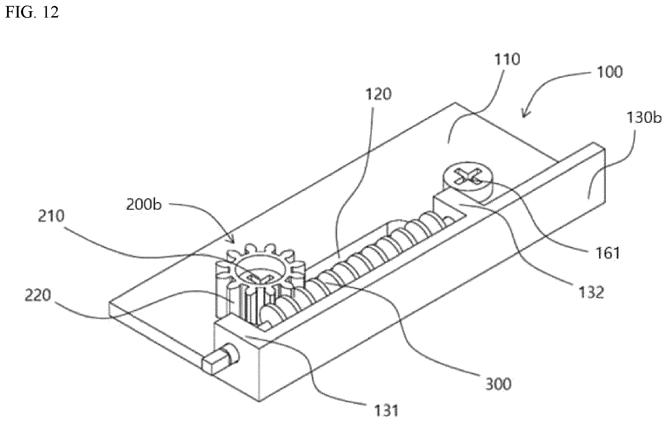

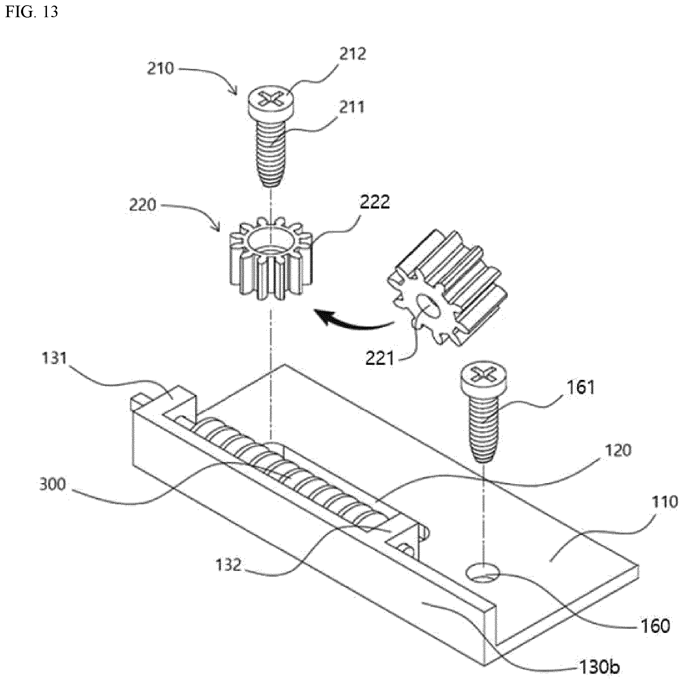

FIG. 12 is a perspective view illustrating a state in which a locking screw engages with a worm in a bone orthopedic device with adjustable spacing according to a second exemplary embodiment of the present invention. FIG. 13 is an exploded view of the bone orthopedic device with adjustable spacing in FIG. 12.

Referring to FIGS. 12 and 13, the bone orthopedic device with adjustable spacing according to the second exemplary embodiment of the present invention includes a plate 100, a locking screw 200b, and a worm 300.

The plate 100 includes a main body 110, a through-portion 120 formed to pass through the main body 110 with a predetermined length, and a first support portion 131 and a second support portion 132 which protrude on a bottom surface, and are spaced apart from each other at one side of the main body 110. The plate 100 is placed across two adjacent bone fragments, a first bone and a second bone, and one surface of the main body 110 of the plate 100 is supported on an upper surface of the first bone and the other surface is supported on an upper surface of the second bone.

The side wall 130b is formed at a predetermined height in the longitudinal direction of the main body 110 at one side of the main body 110. The first support portion 131 and the second support portion 132 are formed in the form of walls extending inward from the side wall 130b. The worm 300 is placed between the side wall 130b and the through-portion 120. A distance between the first support portion 131 and the second support portion 132 is formed to be greater than a length of the through-portion 120. One end portion of the worm 300 is supported by passing through the first support portion 131, and the other end portion of the worm 300 is supported by passing through the second support portion 132.

The through-portion 120 has an oval shape that extends in the longitudinal direction of the main body 110. When one side of the through-portion 120 may be placed at the upper portion of the first bone, and the other side may be placed at the upper portion of the second bone, or a screw is fastened by forming a hole outside the through-portion 120, the through-portion 120 may be placed on only any one of the first and second bones. A width of the through-portion 120 is formed to be larger than a diameter of a body portion 211 of a screw body 210, while being formed to be smaller than a diameter of a head portion 212 of the screw body 210.

The locking screw 200b is inserted into the through-portion 120. The locking screw 200b includes the screw body 210 and the screw head 220 which are enabled to be coupled to each other. An outer peripheral surface sawtooth 222 is formed on an outer peripheral surface of the screw head 222. The outer peripheral surface sawtooth 222 is formed along a circumference of the outer peripheral surface of the screw head 220. **In** the screw head 220, a hollow with a diameter equal to or greater than a diameter of the head portion 212 of the screw body 210 is formed, so the screw body 210 may be inserted into the hollow. In a state where the screw head 220 is placed on the main body 110 of the plate 100 on an upper side of the through-portion 120, the screw body 210 passes through the through-portion 120, so an end portion of the screw body 210 is inserted into the first or second bone.

A catching jaw 221 protruding inward is formed at a lowest adjacent portion of an inner peripheral surface of the screw head 220. While the screw body 210 is inserted into the first or second bone, a lower surface of the head portion 212 of the screw body 210 is caught in the catching jaw 221 of the screw head 220. The head portion 212 of the screw body 210 is supported on the catching jaw 221, while the catching jaw 221 extends inward with a length not to be in contact with the outer peripheral surface of the body portion 211 of the screw body 210. The head portion 212 of the screw body 210 is caught on the catching jaw 221 not to be inserted downward any further.

The worm 300 is supported on the first support portion 131 and the second support portion 132, and the locking screw 200b is used as a worm heel, and a screw thread is formed which engages with the outer peripheral surface sawtooth 222 of the locking screw 200b. The worm 300 is placed between the side wall 130b and the locking screw 200b. The screw thread is formed in a longitudinal direction of the worm 300 with approximately an extended length of the through-portion 120 to correspond to the through-portion 120 of a side portion. The outer peripheral surface sawtooth 222 of the locking screw 200b is configured so that the locking screw 200b may move at a predetermined gear ratio in the longitudinal direction of the through-portion 120 when the screw head 220 rotates with engaging with the screw thread of the worm 300. After the locking screw 200b moves by a predetermined length, reverse rotation of the screw head 220 of the locking screw 200b is prevented by the worm 300, so that the locking screw 200b stops at a position to which the screw head 220 moves.

A motor (not illustrated) may be connected to one or the other side of the worm 300. The motor may rotate the worm 300 in one or the other direction. Further, the motor may be mounted on the plate 100 and designed as an integral form with the plate 100.

Meanwhile, the screw fixing hole 160 spaced apart from the through-portion 120 may be formed in the plate 100. The locking screw 200b may be operated while the plate 100 is fixed to the first or second bone using the fixing screw 161 in the screw fixing hole 160. Accordingly, a fixing force of the plate 100 is increased, enabling a more stable procedure.

The bone orthopedic device with adjustable spacing according to the second exemplary embodiment may be applied to the navigation system for oral and maxillofacial surgery according to the present invention described above as follows.

The display unit 60 displays a plurality of partial images which extracts at least a part of the 3D image of the patient photographed by the **MRI** or CT. The storage unit 40 stores a predetermined target arrival point among the partial images. The calculation unit 50 may calculate an arrival distance between the target arrival point, and the locking screw 200b, and the display unit 60 may display a target arrival point and an arrival distance on the partial image. The motor may rotate the worm 300 so that the locking screw 200b moves up to the target arrival point. Accordingly, when the doctor sets the target arrival point through a preliminary simulation task, the worm 300 automatically rotates to perform the surgery.

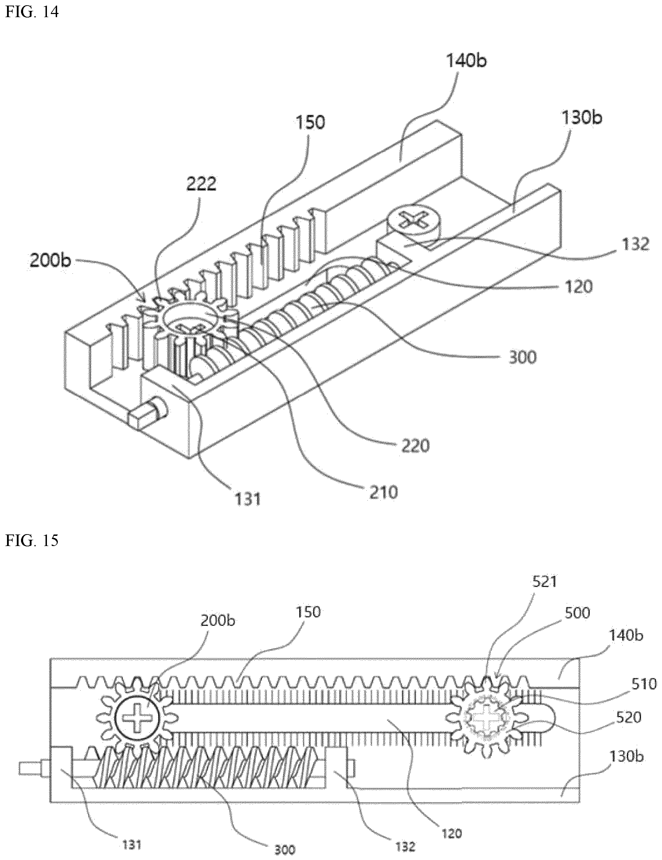

FIG. 14 is a perspective view illustrating a state in which a second side wall is provided in the bone orthopedic device with adjustable spacing according to the second exemplary embodiment of the present invention.

Referring to FIG. 14, the bone orthopedic device with adjustable spacing according to the present invention may further include a second side wall 140b. A plurality of plate sawteeth 150 in which the locking screw 200b engages with the outer peripheral surface sawtooth 222 are formed in a longitudinal direction at an opposite side to the worm 300 on the second side wall 140b. The second side wall 140b is formed at the other side of the plate 100 opposite to the side wall 130b. A spacing between the side wall 130b and the through-portion 120 is formed to be larger than a spacing between the second side wall 140b and the through-portion 120. A distance between the longitudinal axial lines of the second side wall 140b and the through-portion 120 is formed approximately at a radius of a screw head 220 of the locking screw 200b. A height of each of the side wall 130b and the second side wall 140b may be formed approximately at a height of the screw head 220.

The plate sawtooth 150 is formed to extend in a longitudinal direction of an inner surface of the second side wall 140b with a length larger than a length of the through-portion 120. A height of the plate sawtooth 150 may be formed to be substantially equal to a height of the outer peripheral surface sawtooth 222 formed on the outer peripheral surface of the screw head 220.

FIG. 15 is a plan view illustrating an application example, in which the bone orthopedic device with adjustable spacing in FIG. 14 includes the locking screw and the second locking screw.

Referring to FIG. 15, the bone orthopedic device with adjustable spacing according to the second exemplary embodiment of the present invention may include a locking screw 200b and a second locking screw 500. The second locking screw 500 is placed within the through-portion 120, and is placed to be spaced apart from the locking screw 200b. A configuration of the locking crew which is enabled to be locked or unlocked, which is used in the bone orthopedic device with adjustable spacing according to the first exemplary embodiment described above in FIG. 5 may be applied to the second locking screw 500 as it is. The locking screw 200b is placed on the upper side of the first bone, and the second locking screw 500 is placed on the upper side of the second bone.

It is designed so that the worm 300 engages with the locking screw 200b, and the worm 300 does not engage with the second locking screw 500. Further, both the locking screw 200b and the second locking screw 500 are designed to engage the plate sawtooth 150. When the worm 300 rotates while the second locking screw 500 is fixed in position relative to the second bone, the locking screw 200b moves to one or the other side of the longitudinal direction of the through-portion 120, thereby controlling the movement of the first bone. Meanwhile, while the locking screw 200b is fixed in position relative to the first bone, the operator may move the second locking screw 500 to one or the other side of the longitudinal direction of the through-portion 120, thereby controlling the movement of the second bone. **In** detail, the operator may fix the screw body 510 of the second locking screw 500 to the second bone and then rotate the screw head 520 of the second locking screw 500 using a tool, etc. Accordingly, an outer peripheral surface sawtooth 521 provided on the screw head 520 of the second locking screw 500 engages with the plate sawtooth 150, causing the second locking screw 500 to move.

A protection scope of this field is not limited to disclosure and expression of the exemplary embodiment explicitly described above. Further, it is additionally mentioned once again that the protection scope of the present invention cannot be limited due to apparent change or substitution in a technical field to which the present invention belongs.

### [Explanation of Reference Numerals and Symbols]

1: Standard tracked object
2: Reference tracked object
100: Plate
110: Main body
10: Standard tracked object mount
11: Tooth mounting part
12: Standard tracked object fixing member
20: Reference tracked object mount
21: Reference tracked object fixing member
30: Location tracking device
31: Infrared camera
40: Storage unit
50: Calculation unit
60: Display unit
100: Plate
110: Main body
111: Body ratchet sawtooth
120: Through-portion
130a, b: Side wall
131: First support portion
132: Second support portion
140a, b: Second side wall
150: Plate sawtooth
160: Screw fixing hole
161: Fixing screw
200: Locking screw
200a, b: Locking screw
210: Screw body
211: Body portion
211a: Screw thread of body portion
212: Head portion
212a: Locking sawtooth
212b: Insertion groove
220: Screw head
221: Catching jaw
222: Outer peripheral surface sawtooth
223: Inner peripheral surface sawtooth
224: Head ratchet sawtooth
225: Rotating groove
300: Worm
400: Elastic member
500: Second locking screw
s1: Locking area
s2: Locking release area

## Claims

1. A navigation system for oral and maxillofacial surgery, comprising:
a standard tracked object mount which is detachable from teeth of a patient and to which a standard tracked object is mounted;
a bone orthopedic device including a plate, which includes a main body and a through-portion formed in the main body, and a locking screw, which passes through the through-portion, is inserted and fixed to a jaw bone of the patient, and can move left and right along the through-portion;
a reference tracked object mount of which one side is mounted to the locking screw and the other side has a reference tracked object mounted thereto;
a location tracking device that is enabled to track the standard tracked object and the reference tracked object;
a storage unit that stores a 3D image of the patient captured before surgery; and
a calculation unit that matches a coordinate system having the standard tracked object as an origin with a coordinate system of the 3D image, and calculates a position of the locking screw in the matched coordinate system by using a positional relationship between the standard tracked object and the reference tracked object.

2. The navigation system for oral and maxillofacial surgery of claim 1, wherein the reference tracked object is a plurality of infrared markers, and the location tracking device is a plurality of infrared cameras.

3. The navigation system for oral and maxillofacial surgery of claim 1, wherein the reference tracked object is an EM pointer, and the location tracking device includes an EM generation unit and an EM tracker that tracks a location of the EM pointer.

4. The navigation system for oral and maxillofacial surgery of claim 1, further comprising:
a display unit displaying a plurality of partial images extracting at least a portion of the 3D image,
wherein the storage unit stores a target arrival point of the locking screw in the partial image, the calculation unit calculates an arrival distance between the target arrival point and a current location of the locking screw, and the display unit displays the target arrival point and the arrival distance in real time on the partial image.

5. The navigation system for oral and maxillofacial surgery of claim 4, wherein the calculation unit calculates an orientation of the locking screw to calculate an orientation of the bone orthopedic device on the matched coordinate system, and the display unit displays the orientation of the bone orthopedic device.

6. The navigation system for oral and maxillofacial surgery of claim 4, wherein the calculation unit calculates a facial contour change simulation of the patient in the 3D image as the locking screw moves up to the target arrival point, and the display unit displays the 3D image to which the facial contour change simulation of the patient is reflected in real time.

7. The navigation system for oral and maxillofacial surgery of claim 1, wherein the locking screw has a screw body and a screw head that are enabled to be coupled to each other, and an outer peripheral surface sawtooth is formed on an outer peripheral surface of the screw head.

8. The navigation system for oral and maxillofacial surgery of claim 7, wherein the plate includes a side wall formed at one side of the main body, and a plurality of plate sawteeth is formed at the through-portion on the side wall,
the screw body includes a body portion having a screw thread formed on an outer peripheral surface thereof, and a head portion coupled onto the body portion and having a locking sawtooth formed on an outer peripheral surface thereof, and
the outer peripheral surface sawtooth of the screw head engages with the plate sawtooth, and an inner peripheral surface sawtooth engaged with the locking sawtooth of the head portion of the screw body is formed on a portion of an inner peripheral surface of the screw head.

9. The navigation system for oral and maxillofacial surgery of claim 7, wherein the screw head is supported on an upper surface of the main body of the plate at an upper side of the through-portion.

10. The navigation system for oral and maxillofacial surgery of claim 7, wherein an inner peripheral surface of the screw head includes a locking area in which an inner peripheral surface sawtooth is formed, and a locking release area in the locking area, and
the locking area is formed to extend at a predetermined height in the inner peripheral surface of the screw head.

11. The navigation system for oral and maxillofacial surgery of claim 7, wherein a catching jaw protruding inward is formed on a lower end of an inner peripheral surface of the screw head, and a lower surface of the head portion of the screw body is enabled to be caught on the catching jaw.

12. The navigation system for oral and maxillofacial surgery of claim 7, further comprising:
an elastic member which is placed between a lower surface of the screw head, and the main body, and of which one side is bent toward the lower surface of the screw head.

13. The navigation system for oral and maxillofacial surgery of claim 12, wherein the elastic member is a spring washer.

14. The navigation system for oral and maxillofacial surgery of claim 7, further comprising:
a head ratchet sawtooth formed on a lower surface of the screw head, and a body ratchet sawtooth formed on an upper surface of the main body and engaged with the head ratchet sawtooth.

15. The navigation system for oral and maxillofacial surgery of claim 7, wherein the locking screw includes a first locking screw, and a second locking screw inserted into the through-portion and placed to be spaced apart from the first locking screw.

16. The navigation system for oral and maxillofacial surgery of claim 7, wherein a screw fixing hole spaced apart from the through-portion and inserted with a screw is formed on the plate.

17. The navigation system for oral and maxillofacial surgery of claim 1, wherein the plate includes a first support portion and a second support portion which protrude from a bottom surface of the main body, and are spaced from each other, and
includes a worm supported by the first support portion and the second support portion, and engaged with an outer peripheral surface sawtooth of the locking screw by using the locking screw as a worm heel.

18. The navigation system for oral and maxillofacial surgery of claim 17, wherein one end portion of the worm is supported by passing through the first support portion, and the other end portion of the worm is supported by passing through the second support portion.

19. The navigation system for oral and maxillofacial surgery of claim 17, further comprising:
a motor connected to the worm, and rotating the worm in one direction or the other direction.

20. The navigation system for oral and maxillofacial surgery of claim 19, further comprising:
a display unit displaying a plurality of partial images extracting at least a portion of the 3D image,
wherein the storage unit stores a predetermined target arrival point among the partial images, the calculation unit calculates an arrival distance between the target arrival point and the locking screw, the display unit displays the target arrival point and the arrival distance on the partial image, and the motor rotates the worm so that the locking screw moves up to the target arrival point.

21. The navigation system for oral and maxillofacial surgery of claim 17, wherein the plate further includes a side wall formed at the other side of the main body, and a plurality of plate sawteeth engaged with the outer peripheral surface sawtooth of the locking screw is formed at an opposite side to the worm on the side wall.

22. The navigation system for oral and maxillofacial surgery of claim 21, further comprising:
a second locking screw inserted into the through-portion, and placed to be spaced apart from the locking screw,
wherein the second locking screw is enabled to move left and right along the through-portion by engaging with the plate sawtooth.
